# EUROPÄISCHE PATENTSCHRIFT

(11) **EP 0 600 339 B1**
(45) Veröffentlichungstag und Bekanntmachung des Hinweises auf die Patenterteilung: **10.01.1996**
(21) Anmeldenummer: 93118750.4
(22) Anmeldetag: 22.11.1993
(51) Int. Cl.: C07C 313/04

(54) **Verfahren zur Herstellung von Natriumsalzen nitrogruppenhaltiger aromatischer Sulfinsäuren**
Method for the preparation of sodium salts of aromatic sulphinic acids containing nitro groups
Procédé pour la préparation des sels de sodium des acides sulfiniques aromatiques contenant des groupes nitro

(30) Priorität: 03.12.1992 DE 4240708
(43) Veröffentlichungstag der Anmeldung: 08.06.1994
(73) Patentinhaber: BAYER AG, D-51368 Leverkusen (DE)
(72) Erfinder: Silber, Gunter, Dr., D-51061 Köln (DE); Schade, Gerold, Dr., D-51375 Leverkusen (DE); Thiel, Udo, D-51375 Leverkusen (DE)

(56) Entgegenhaltungen:
- DE-A- 2 056 758
- DE-A- 4 022 477
- GB-A- 2 155 469

## Beschreibung

Die Erfindung betrifft ein Verfahren zur Herstellung von Natriumsalzen nitrogruppenhaltiger aromatischer Sulfinsäuren durch Umsetzung der entsprechenden Sulfonsäurechloride mit Alkalisulfit in Gegenwart von Natronlauge.

Aus Natriumsalzen nitrogruppenhaltiger aromatischer Sulfinsäuren kann man beispielsweise durch Alkylierung die entsprechenden Sulfone erhalten, die ihrerseits als Zwischenprodukte zur Herstellung von Reaktivfarbstoffen dienen können.

Es ist bekannt, Natriumsulfinate herzustellen, indem man zu einer wäßrigen Natriumsulfit-Lösung im Verlauf von mehreren Stunden bei 10 bis 70°C das Sulfonsäurechlorid zugibt und gleichzeitig Natronlauge hinzufügt, um das Reaktionsmedium alkalisch zu halten (FIAT Final Report 949, S. 23 bis 24). Bei einem anderen Verfahren werden zu einem Gemisch aus Natriumbisulfit, Natronlauge, Phosphorsäure und Wasser im Verlauf von 2 Stunden bei 60°C das Sulfonsäurechlorid und gleichzeitig Natronlauge hinzufügt (DE-OS 33 02 647, Beispiel 17).

Nachteilig bei diesen Verfahren ist, daß so hergestellte Natriumsulfinate in einer Ausbeute von nur ca. 80 % anfallen und stets 4 bis 6 Gew.-% der entsprechenden Natriumsulfonate enthalten, welche in einer Nebenreaktion durch Hydrolyse der Sulfonsäurechloride und anschließende Neutralisation der erhaltenen Sulfonsäuren entstehen.

Dieser Gehalt an Natriumsulfonaten ist unerwünscht, da er einerseits die Ausbeute an gewünschtem Natriumsulfinat herabsetzt und andererseits bei der Weiterverarbeitung der Sulfinate in wäßrigem Milieu zu einer zusätzlichen Abwasserbelastung führen kann.

Es wurde nun gefunden, daß sich durch geeignete Wahl der Reaktionsbedingungen die Natriumsalze nitrogruppenhaltiger aromatischer Sulfinsäuren in ausgezeichneter Ausbeute mit hoher Reinheit herstellen lassen.

Gegenstand der Erfindung ist daher ein Verfahren zur Herstellung von Verbindungen der Formel 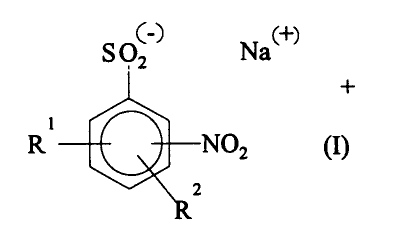
worin R¹ und R² unabhängig voneinander Wasserstoff, C₁-C₆-Alkyl, C₅-C₇-Cycloalkyl, Halogen, C₆-C₁₂-Aryl, C₆-C₁₈-Aralkyl, C₁-C₆-Alkoxy, C₆-C₁₂-Aryloxy, Amino, C₂-C₅-Acylamino oder Carboxy bedeuten,
durch Umsetzung von Sulfonsäurechloriden der Formel 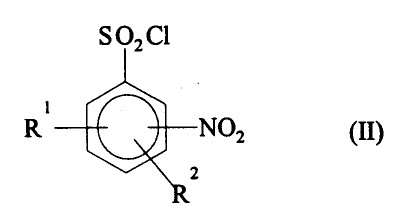
worin R¹ und R² die obengenannten Bedeutungen haben,
mit Alkalisulfit in Gegenwart von Natronlauge, dadurch gekennzeichnet, daß man das Sulfonsäurechlorid (II) in eine wäßrige Alkalisulfitlösung einträgt, die eine Temperatur von -10 bis 45°C, vorzugsweise -5 bis 40°C, und einen pH-Wert von 6 bis 10, vorzugsweise 6 bis 9, aufweist, die Suspension - sofern ihre Temperatur über 0°C beträgt - auf -10 bis 0°C, vorzugsweise -5 bis 0°C, abkühlt und dann Natronlauge so zudosiert, daß die Temperatur während mindestens 80 %, vorzugsweise mindestens 90 % der Umsetzung 0°C nicht überschreitet. Anschließend kann zwecks Nachreaktion bzw. Bildung des Natriumsalzes die Temperatur auf 10 bis 80, vorzugsweise 15 bis 60°C angehoben werden, wobei durch Zugabe von Natronlauge der pH-Wert vorzugsweise auf 8-10, insbesondere 8-9, gehalten wird.

Die Alkylreste (R¹ bzw. R²) können linear oder verzweigt sein. Sie enthalten vorzugsweise 1 bis 4 Kohlenstoffatome. Beispiele geeigneter Alkylreste sind Methyl, Ethyl, Propyl, Isopropyl, Butyl, Isobutyl, Pentyl, Isopentyl, Hexyl und Isohexyl.

Bevorzugte Cycloalkylreste (R¹ bzw. R²) umfassen Cyclopentyl und insbesondere Cyclohexyl.

Halogen (R¹ bzw. R²) umfaßt Iod und vorzugsweise Fluor, Chlor und Brom.

Aryl (R¹ bzw. R²) umfaßt vorzugsweise Phenyl und Tolyl.

Als Aralkylreste (R¹ bzw. R²) kommen beispielsweise solche mit 6 bis 18 Kohlenstoffatomen, deren aliphatischer Teil 1 bis 6 Kohlenstoffatome enthält, und deren aromatischer Teil einen Rest aus der Benzolreihe darstellt, infrage. Bevorzugte Beispiele umfassen β-Phenyl-ethyl, γ-Phenyl-propyl und β-Phenyl-n-hexyl, insbesondere Benzyl.

Alkoxyreste (R¹ bzw. R²) umfassen lineare und verzweigte Reste mit vorzugsweise 1 bis 4 C-Atomen, wie z.B. Methoxy, Ethoxy, Propoxy, Isopropoxy, Butoxy und Isobutoxy.

Aryloxy bedeutet vorzugsweise Phenoxy und Acylamino bedeutet vorzugsweise Acetylamino.

Als Ausgangsverbindungen bevorzugte Sulfonsäurechloride entsprechen der Formel (II), worin R¹ und R² unabhängig voneinander Wasserstoff, einen Alkylrest mit 1 bis 4 Kohlenstoffatomen, Fluor, Chlor, Brom, Phenyl, Methoxy, Ethoxy, Phenoxy oder Acetylamino bedeuten, wie z.B.
o-Nitrobenzolsulfochlorid,
m-Nitrobenzolsulfochlorid,
p-Nitrobenzolsulfochlorid,
2-Methyl-5-nitro-benzolsulfochlorid,
4-Methyl-3-nitro-benzolsulfochlorid,
2-Ethyl-5-nitro-benzolsulfochlorid,
2,4-Dimethyl-3-nitro-benzolsulfochlorid,
2-Chlor-5-nitro-benzolsulfochlorid,
4-Chlor-3-nitro-benzolsulfochlorid,
2-Methoxy-5-nitro-benzolsulfochlorid,
4-Methoxy-3-nitro-benzolsulfochlorid,
4-Acetylamino-3-nitro-benzolsulfochlorid.

Der Begriff "Alkalisulfit" im Sinne der Erfindung schließt auch Alkalihydrogensulfite ein.

Als wäßrige Alkalisulfitlösung kommen beispielsweise 15 bis 40 gew.-%ige, wäßrige Lösungen eines Alkalisulfits oder Alkalihydrogensulfits infrage. Bevorzugt sind Lösungen von Natriumsulfit oder Natriumhydrogensulfit. Pro Gewichtsteil Sulfonsäurechlorid kann man beispielsweise 3 bis 10 Gewichtsteile der Alkalisulfitlösung einsetzen. Um einen pH-Wert im Bereich von 6 bis 10 einzustellen, kann es gegebenenfalls erforderlich sein, eine Lauge (z.B. Natronlauge) oder eine Säure (z.B. Salzsäure) zuzusetzen. Beim Einsatz von Hydrogensulfiten sind im allgemeinen Laugenzusätze erforderlich.

Die Natronlauge kann beispielsweise eine Konzentration von 30 bis 60 Gew.-% aufweisen. Bevorzugt ist Natronlauge mit einer Konzentration von 45 bis 50 Gew.-%. Die Geschwindigkeit des Natronlaugezulaufs wird so geregelt, daß die freiwerdende Reaktionswärme abgeführt werden kann, ohne daß das Reaktionsgemisch eine Temperatur von 0°C überschreitet.

Dagegen ist es nicht erforderlich, während der Natronlaugedosierung einen bestimmten pH-Wert einzuhalten. Vielmehr kann der pH-Wert während der Reaktion in weiten Grenzen, beispielsweise zwischen 3 und 7, schwanken.

Nach beendeter Umsetzung kann das Reaktionsgemisch auf an sich bekannte Weise aufgearbeitet werden: Beispielsweise kann man so vorgehen, daß man das Reaktionsgemisch auf 40 bis 95°C, vorzugsweise 40 bis 70°C, erwärmt, wobei gegebenenfalls durch Zusatz von Wasser und Natronlauge das Reaktionsgemisch verdünnt und der pH-Wert auf beispielsweise 8 bis 12, vorzugsweise 8 bis 11,5 gestellt wird.

Nach einer sich gegebenenfalls anschließenden Klärfiltration kann das Produkt durch Abkühlen, gegebenenfalls unter Zusatz von Natriumchlorid, ausgefällt und in bekannter Weise, z.B. durch Filtration, isoliert werden.

Erfindungsgemäß hergestellte, nitrogruppenhaltige, aromatische Sulfinsäure-Natriumsalze enthalten im allgemeinen weniger als 1 Gew.-% der entsprechenden Sulfonsäure und fallen im allgemeinen in einer Ausbeute von deutlich über 90 % d. Th. an.

### Beispiele

### Beispiel 1

Zu einer Lösung von 184 g Natriumhydrogensulfit in 649 ml Wasser wurden 67 ml 50 gew.-%ige Natronlauge zugegeben, wobei sich ein pH-Wert von 6,4 einstellt. Danach wurde auf -5°C abgekühlt und 2-Chlor-5-nitro-benzolsulfonsäurechlorid in wasserfeuchter Form (Wassergehalt 20 Gew.-%, 256 g 2-Chlor-5-nitro-benzolsulfonsäurechlorid 100 %ig) auf einmal hinzugefügt.

Danach wurden 100 ml 50 gew.-%ige Natronlauge so zulaufen gelassen, daß die Temperatur durch Kühlung im Bereich von -5 bis 0°C gehalten werden konnte. Danach wurde mit Natronlauge ein pH-Wert von 8 bis 9 eingestellt und 30 Minuten bei -5 bis 0°C nachgerührt. Durch Erwärmen auf 15°C bei gleichzeitigem Halten des pH-Wertes im Bereich 8-9 durch Zugabe von 18 ml 50 gew.-%ige Natronlauge wurde das Produkt in das Natriumsalz überführt. Man ließ 30 Minuten nachrühren.

Abschließend wurde auf 60°C erwärmt, 2 g Filtrierhilfsmittel (®Celite) zugesetzt und filtriert.

Schließlich wurden 440 g Steinsalz hinzugefügt, auf 20°C abgekühlt und durch Filtration das 2-Chlor-5-nitro-benzolsulfinsäure-Natriumsalz in einer Ausbeute von 95 % mit einem Gehalt an 2-Chlor-5-nitro-benzolsulfonsäure-Natriumsalz von 0,8 Gew.-% in Form eines wasserfeuchten Filterkuchens (Wassergehalt 50 Gew.-%) isoliert.

### Beispiel 2 (Vergleichsbeispiel)

Zu einer Lösung von 184 g Natriumhydrogensulfit in 649 ml Wasser wurden 70 ml 50 gew.-%ige Natronlauge zugegeben, wobei sich ein pH-Wert von 6,6 einstellt. Danach wurde auf 20°C abgekühlt und 2-Chlor-5-nitro-benzolsulfonsäurechlorid in wasserfeuchter Form (Wassergehalt 20 Gew.-%, 256 g 2-Chlor-5-nitro-benzolsulfonsäurechlorid 100 %ig) auf einmal hinzugefügt.

Danach wurden 112 ml 50 gew.-%ige Natronlauge so zulaufen gelassen, daß die Temperatur durch Kühlung im Bereich von 20 bis 22°C gehalten werden konnte.

Danach wurde mit Natronlauge ein pH-Wert von 10,5 eingestellt und 30 Minuten bei 20 bis 22°C nachgerührt. Während des Nachrührens wurde durch Zugabe von weiteren 2 ml 50 gew.-%iger Natronlauge der pH-Wert im Bereich 10 bis 11 gehalten. Abschließend wurde auf 60°C erwärmt und filtriert. Schließlich wurden 440 g Steinsalz hinzugefügt, auf 20°C abgekühlt und durch Filtration das 2-Chlor-5-nitro-benzolsulfinsäure-Natriumsalz in einer Ausbeute von 82,2 % d. Th. in Form eines wasserfeuchten Filterkuchens (Wassergehalt 50 Gew.-%) isoliert.

### Beispiel 3 (Vergleichsbeispiel)

Eine Natriumsulfitlösung, bestehend aus 2 400 kg Natriumbisulfit (30 gew.-%ig) sowie 5 700 l Wasser und 450 l Natronlauge (50 gew.-%ig) wurden vorgelegt. Die Lösung wurde mit Natronlauge auf pH 8,5 gestellt und auf 10°C gekühlt. Nun wurden bei 10 bis 18°C innerhalb von 6 Stunden 2 000 kg 2-Chlor-5-nitro-benzolsulfonsäurechlorid in wasserfeuchter Form (Wassergehalt 30 Gew.-%) und gleichzeitig 574 l 50 gew.-%ige Natronlauge eingetragen, wobei der pH-Wert durchgehend bei etwa 8 bis 9 gehalten wurde. Es wurde noch 120 Minuten nachgerührt und dann wie in Beispiel 1 beschrieben aufgearbeitet.

Es wurde 2-Chlor-5-nitro-benzolsulfinsäure-Natriumsalz in einer Ausbeute von 83 % d.Th. erhalten.

### Beispiel 4

Zu einer Lösung von 194 g Natriumhydrogensulfit in 666 ml Wasser wurden 70 ml 50 gew.-%ige Natronlauge zugegeben, wobei sich ein pH-Wert von 6,4 einstellt. Danach wurde auf -5°C abgekühlt und 2-Methyl-5-nitro-benzolsulfonsäurechlorid in wasserfeuchter Form (Wassergehalt 20 Gew.-%, 236 g 2-Methyl-5-nitro-benzolsulfonsäurechlorid 100 %ig) auf einmal hinzugefügt.

Danach wurden 120 ml 50 gew.-%ige Natronlauge so zulaufen gelassen, daß die Temperatur durch Kühlung im Bereich -5 bis 0°C gehalten werden konnte. Danach wurde mit 28 ml 50 gew.-%iger Natronlauge ein pH-Wert von 8 bis 9 eingestellt und 30 Minuten bei -5 bis 0°C nachgerührt. Dann wurde zum Aussalzen auf 25°C erwärmt und nochmals 30 Minuten nachgerührt.

Schließlich wurden 440 g Steinsalz hinzugefügt, auf 20°C abgekühlt und durch Filtration das 2-Methyl-5-nitro-benzolsulfinsäure-Natriumsalz in einer Ausbeute von 93 % mit einem Gehalt an 2-Methyl-5-nitro-benzolsulfonsäure von 0,8 Gew.-% in Form eines wasserfeuchten Filterkuchens (Wassergehalt 50 Gew.-%) isoliert.

## Patentansprüche

1. Verfahren zur Herstellung von Verbindungen der Formel 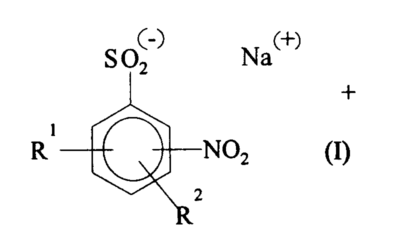 worin R¹ und R² unabhängig voneinander Wasserstoff, C₁-C₆-Alkyl, C₅-C₇-Cycloalkyl, Halogen, C₆-C₁₂-Aryl, C₆-C₁₈-Aralkyl, C₁-C₆-Alkoxy, C₆-C₁₂-Aryloxy, Amino, C₂-C₅-Acylamino oder Carboxy bedeuten,
durch Umsetzung von Sulfonsäurechloriden der Formel 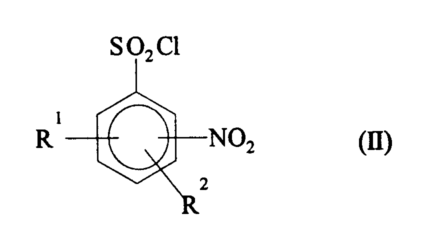 worin R¹ und R² die obengenannten Bedeutungen haben, mit Alkalisulfit in Gegenwart von Natronlauge, dadurch gekennzeichnet, daß man das Sulfonsäurechlorid (II) in eine wäßrige Alkalisulfitlösung einträgt, die eine Temperatur von -10 bis 45°C und einen pH-Wert von 6 bis 10 aufweist, die Suspension - sofern ihre Temperatur über 0°C beträgt - auf -10 bis 0°C abkühlt, dann die Natronlauge so zudosiert, daß die Temperatur während mindestens 80 % der Umsetzung 0°C nicht überschreitet.

2. Verfahren nach Anspruch 1, wonach man das Sulfonsäurechlorid (II) in eine wäßrige Alkalisulfitlösung einträgt, die eine Temperatur von -5 bis 40°C aufweist.

3. Verfahren nach Anspruch 1, wonach man das Sulfonsäurechlorid (II) in eine wäßrige Alkalisulfitlösung einträgt, die einen pH-Wert von 6 bis 9 aufweist.

4. Verfahren nach Anspruch 1, wonach man die Suspension - sofern ihre Temperatur über 0°C beträgt - auf -5 bis 0°C abkühlt und dann die Natronlauge zudosiert.

5. Verfahren nach Anspruch 1, wonach man die Natronlauge so zudosiert, daß die Temperatur während mindestens 90 % der Umsetzung 0°C nicht überschreitet.

## Claims

1. Process for the preparation of compounds of the formula 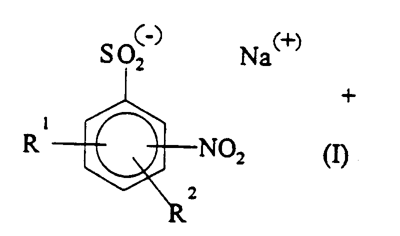 in which R¹ and R² independently of one another are hydrogen, C₁-C₆-alkyl, C₅-C₇-cycloalkyl, halogen, C₆-C₁₂-aryl, C₆-C₁₈-aralkyl, C₁-C₆-alkoxy, C₆-C₁₂-aryloxy, amino, C₂-C₅-acylamino or carboxyl,
by the reaction of sulphonyl chlorides of the formula 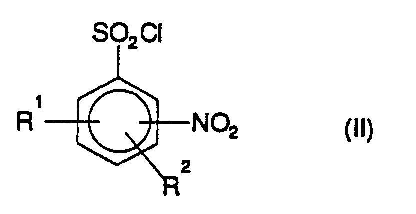 in which R¹ and R² are as defined above,
with alkali metal sulphite in the presence of sodium hydroxide solution, characterised in that the sulphonyl chloride (II) is introduced into an aqueous alkali metal sulphite solution which is at a temperature of -10 to 45°C and has a pH of 6 to 10, the suspension - if its temperature is above 0°C - is cooled to -10 to 0°C and the sodium hydroxide solution is then metered in in such a way that the temperature does not exceed 0°C for at least 80% of the reaction time.

2. Process according to Claim 1, wherein the sulphonyl chloride (II) is introduced into an aqueous alkali metal sulphite solution which is at a temperature of -5 to 40°C.

3. Process according to Claim 1, wherein the sulphonyl chloride (II) is introduced into an aqueous alkali metal sulphite solution which has a pH of 6 to 9.

4. Process according to Claim 1, wherein the suspension - if its temperature is above 0°C - is cooled to -5 to 0°C and the sodium hydroxide solution is then metered in.

5. Process according to Claim 1, wherein the sodium hydroxide solution is metered in in such a way that the temperature does not exceed 0°C for at least 90% of the reaction time.

## Revendications

1. Procédé pour la préparation des composés de formule 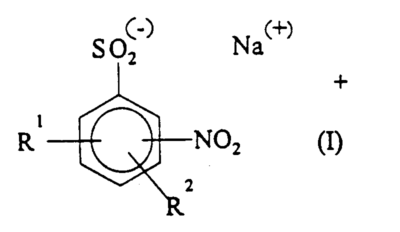 dans laquelle R¹ et R² représentent chacun, indépendamment l'un de l'autre, l'hydrogène, un groupe alkyle en C₁-C₆, cycloalkyle en C₅-C₇, un halogène, un groupe aryle en C₆-C₁₂, aralkyle en C₆-C₁₈, alcoxy en C₁-C₆, aryloxy en C₆-C₁₂, amino, acylamino en C₂-C₅ ou carboxy,
par réaction de chlorures d'acides sulfoniques de formule 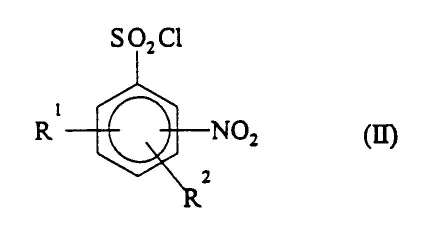 dans laquelle R¹ et R² ont les significations indiquées ci-dessus, avec un sulfite alcalin en présence de lessive de soude, caractérisé en ce que l'on introduit le chlorure d'acide sulfonique II dans une solution aqueuse du sulfite alcalin qui est à une température de -10 à 45°C et présente un pH de 6 à 10, on refroidit la suspension - lorsque sa température est supérieure à 0°C - à un niveau de -10 à 0°C puis on ajoute la lessive de soude à un débit tel que la température, durant au moins 80 % du temps de réaction, ne dépasse pas 0°C.

2. Procédé selon revendication 1, selon lequel on introduit le chlorure d'acide sulfonique II dans une solution aqueuse de sulfite alcalin à une température comprise entre -5 et 40°C.

3. Procédé selon revendication 1, selon lequel on introduit le chlorure d'acide sulfonique II dans une solution aqueuse de sulfite alcalin à un pH de 6 à 9.

4. Procédé selon revendication 1, selon lequel on refroidit la suspension - lorsque sa température est supérieure à 0°C - à un niveau de -5°C à 0°C puis on ajoute la lessive de soude.

5. Procédé selon revendication 1, selon lequel on ajoute la lessive de soude à un débit tel que la température, durant au moins 90 % du temps de réaction, ne dépasse pas 0°C.
